# NOUVEAU FASCICULE DE BREVET EUROPEEN

(11) **EP 1 034 161 B2**
(45) Date de publication et mention de la décision concernant l'opposition: **15.03.2006**
(45) Mention de la délivrance du brevet: 18.09.2002
(21) Numéro de dépôt: 98951634.9
(22) Date de dépôt: 16.11.1998
(51) Int. Cl.: C07C 69/013, C07C 69/63, C07C 69/708, C11B 9/00, A23G 3/00, A23L 1/226

(54) **NOUVEAUX COMPOSES DERIVES DU MENTHOL ET LEUR UTILISATION EN TANT QU'AGENT RAFRAICHISSANT**
MENTHOLDERIVATE UND IHRE ANWENDUNG ALS ERFRISCHUNGSMITTEL
NOVEL COMPOUNDS DERIVED FROM MENTHOL AND USE AS REFRESHING AGENT

(30) Priorité: 27.11.1997 CH 274397
(43) Date de publication de la demande: 13.09.2000
(73) Titulaire: FIRMENICH SA, 1211 Genève 8 (CH)
(72) Inventeur: FREROT, Eric, F-74100 Ville-la-Grand (FR); VAN BEEM, Nicole, CH-1195 Dully (CH)
(74) Mandataire: Salvaterra-Garcia, Maria de Lurdes
(86) Numéro de dépôt international: PCT/IB1998/001821
(87) Numéro de publication internationale: WO 1999/028288

(56) Documents cités:
- DE-A- 2 022 369
- DE-C- 191 547
- FR-A- 2 577 922
- US-A- 3 830 930
- US-A- 4 766 153
- US-A- 5 266 592
- M.H.PALMER ET AL.: "Partial Asymmetric Synthesis of beta-Hydroxy-acids.Part 1. Beta-Hydroxy-beta-phenylbutyric Acid" JOURNAL OF THE CHEMICAL SOCIETY.,1960, pages 931-938, XP002091888 LETCHWORTH GB
- JOAN A.REID ET AL.: "Partial Asymmetric Synthesis in a Reformatsky Reaction" JOURNAL OF THE CHEMICAL SOCIETY.,1949, pages 3365-3368, XP002091889 LETCHWORTH GB
- Pharmazeutische Zeitung, 21, 13.03.1907, p. 212-213
- Rivista Italiana EPPOS, 1995, Spezialausgabe 31.08.-02.09.95, p. 111-115
- Berichte Schimmel, 1892, p. 50-51
- Berichte Schimmel, 1893, p. 58-59
- Perfume and Flavor Chemicals II, S. Arctander, 1969, composés 1840, 1845, 1852
- Berichte Schimmel, 1897, p. 92-93
- Die Aetherischen Oele, E. Gildemeister et al, Verlag von Schimmel & Co. 1910, p. 404-405
- The Essential Oils, vol. II, E. Guenther, D. Van Nostrand Company, New York, 1952, p. 224-226
- Pharmazeutische Zentralhalle, 48, 1907, p. 192
- Roempp, Lexikon Chemie Online, "Menthol"
- Die moderne Parfuemerie, Verlag fuer chem. Industrie, 1904, p. 12, 204, 207

## Description

### Domaine technique

La présente invention a trait à des composés nouveaux ayant un effet rafraîchissant sur la peau ou les membranes muqueuses humaines, notamment les muqueuses buccales et pituitaires. Plus particulièrement, la demande concerne des composés de formule 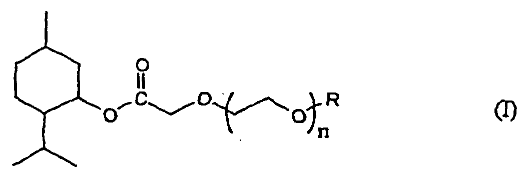 dans laquelle R=H ou CH₃ et n est un nombre entier de 1 à 4, et leur utilisation en tant qu'agents rafraîchissants.

### Technique antérieure

Parmi les composés de structure similaire à celle des composés de formule (I), le méthoxyacétate de 3-p-menthanyl (n=0 et R=CH₃) possède une structure connue. En effet celui-ci est décrit par le brevet DE 191547 qui divulgue le clivage dudit composé dans le but de libérer le menthol. Ce document ne comporte en revanche aucune mention ni même suggestion concernant d'éventuelles propriétés organoleptiques (goût ou odeur) du composé.

Par ailleurs, l'art antérieur décrit un grand nombre de composés, d'origine naturelle ou synthétique, dont on a observé un effet rafraîchissant sur la peau ou les muqueuses humaines, le composé le plus connu étant le (-)-menthol qui se trouve dans la nature dans l'huile de menthe, notamment de *mentha arvensis* L et *mentha viridis L.*

Parmi le grand nombre de publications dans le domaine des agents rafraîchissants synthétiques, et notamment de ceux dérivés du menthol, il convient de citer la demande DE-OS-2608226 qui décrit certains esters du menthol avec des acides carboxyliques hydroxylés, par exemple l'acide glycolique, l'acide β-hydroxybutyrique ou l'acide α-hydroxycaprylique, mais en particulier l'ester de l'acide lactique.

La demande EP A-507190 décrit des agents rafraîchissants qui sont des acétals de certaines cétones, en particulier le glycérol cétal de la 1-menthone.

La demande EP-A-583651 divulgue un autre groupe d'agents rafraîchissants qui sont dérivés du menthol, à savoir des carbonates, carbamates et thiocarbamates asymétriques du menthol, en particulier les carbonates de l'éthylèneglycoi et du propane-1,2-diol du menthol.

Pour être approprié en tant qu'agent rafraîchissant, un composé doit satisfaire à certaines exigences. En premier lieu, le composé ne doit pas avoir un effet irritant sur la peau ou, en particulier, les muqueuses, ce qui ne permettrait pas une utilisation dans certaines applications, par exemple celles dans lesquelles ledit composé est utilisé en grande quantité et/ou le produit d'application peut entrer en contact avec certaines parties sensibles des muqueuses. Pour les raisons exposées ci-dessus, l'utilisation du menthol est limitée, et de même pour d'autres composés qui se révèlent peu appropriés à une utilisation dans certains produits.

De plus, pour de nombreuses applications, il est souhaitable que l'effet rafraîchissant soit d'une durée prolongée, de sorte que cet effet puisse être perçu encore quelques minutes après que l'agent actif ne soit plus en contact avec la peau ou les muqueuses. Le menthol étant d'une grande volatilité, il ne remplit pas cette condition, malgré son effet rafraîchissant prononcé.

Dans la plupart des applications, il est en plus souhaitable de disposer d'agents rafraîchissants qui n'ont pas une forte odeur, comme c'est le cas pour le menthol qui a l'odeur typique et prononcée de la menthe poivrée.

Finalement, un agent rafraîchissant ne doit pas avoir un goût désagréable pour qu'une application dans le domaine des arômes soit possible. Une fois encore, le menthol dispose d'un goût amer prononcé quand il est utilisé à haute concentration.

En résumé, on peut dire que le menthol en particulier, mais aussi les autres composés ayant un effet rafraîchissant et mentionnés ci-dessus, remplissent, certes, certaines des conditions exposées plus haut, recherchées dans les produits rafraîchissants ("cooling agents"), mais on recherche toujours des composés nouveaux dont les propriétés permettent d'obtenir des effets originaux, et de préférence avantageux, dans ce domaine.

### Exposé de l'invention

Nous avons maintenant synthétisé une nouvelle classe de composés qui répond à la formule générale 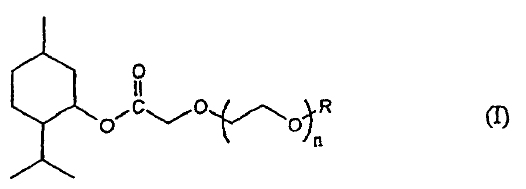 dans laquelle R=H ou CH₃ et n est un nombre entier de 1 à 4.

Par ailleurs, nous avons pu établir que les composés de formule (I) disposent de toutes les propriétés souhaitées d'un agent rafraîchissant, à savoir :
- il ne s'agit pas de composés irritants
- l'effet rafraîchissant est prononcé et de longue durée
- les composés n'ont pas une odeur forte
- le goût des composés est neutre, pouvant renforcer le goût typique des ingrédients dans les applications arômes.

La classe des composés de formule (I) n'a pas le goût typique du menthol, mais un goût plus neutre qui peut varier selon la longueur de la chaîne liée à la fonction carboxylique, la nature du substituant terminal de ladite chaîne (H ou CH₃) ou encore la configuration isomérique du menthol utilisé comme produit de départ pour la préparation des composés (I). En effet, la stéréoisomérie des composés (I) est dictée par celle du menthol de départ et on peut ainsi obtenir tous les stéréoisomères des composés (I) correspondant à ceux du menthol. Parmi ces stéréoisomères, les composés (I) dérivés du (-)-menthol sont particulièrement appréciés, et préférés selon l'invention, pour leur effet rafraîchissant prononcé lorsqu'ils sont utilisés dans les applications décrites plus loin.

Comme il ressort de la formule (I), les composés selon l'invention peuvent porter un groupe alcool ou méthoxy en position terminale de la chaîne. Dans le contexte de la présente invention, on préfère utiliser des composés ayant un groupe méthoxy en position terminale.

Parmi lesdits composés, on préfère ceux obéissant à la formule (I) dans lesquels n=1, à savoir le 3,6-dioxaheptanoate de (-)-menthyle, ou 3,6-dioxaheptanoate de (1R,3R,4S)-3-menthyle. Le 3,6-dioxaheptanoate de (-)-menthyle possède une note amère. Ce composé est très apprécié dans le domaine des arômes et, selon l'application envisagée, on peut l'utiliser seul ou en mélange avec méthoxyacétate de (1R,3R,4S) qui possède lui une note gustative de tête fruitée rapelant celle de l'acétate de menthyle. Par exemple, on utilisera le 3,6-dioxaheptanoate de (-)-menthyle dans les cas où le goût amer est bienvenu, par exemple dans des produits comestibles à base d'agrumes.

Selon un autre mode d'exécution de l'invention, on utilise un mélange de méthoxyacétate de (-)-menthyle et de 3,6-dioxaheptanoate de (-)-menthyle. Dans ces mélanges, les deux composants peuvent être présents dans des proportions relatives très variées qui sont fonction de l'effet que l'on désire obtenir. En effet, l'utilisation de ces mélanges permet de supprimer certaines caractéristiques gustatives de l'un ou l'autre de ces deux composés, lorsqu'elles sont moins appréciées dans certaines applications, tout en renforçant l'effet de fraîcheur. Nous avons constaté que cet effet synergique entre les deux composés se manifestait au mieux avec les mélanges contenant des quantités pondérales similaires des deux composés, c'est-à-dire environ 50% en poids de chacun, ces mélanges étant par conséquent préférés selon l'invention.

Cependant, il est clair que l'invention concerne également les mélanges des deux composés où l'on fait varier la proportion de 3,6-dioxaheptanoate de methyl jusqu'à 100% en poids du mélange.

Par ailleurs, ce composé se prête également à des applications autres que dans le domaine arômes, par exemple dans des produits de soins corporels ou cosmétiques.

Ainsi, les composés de l'invention peuvent être utilisés dans tous les domaines dans lesquels on désire conférer un effet rafraîchissant aux produits dans lesquels ils sont incorporés. A titre d'exemple, il convient de citer des boissons, telles que des jus de fruits, des limonades ou du thé froid, des glaces et des sorbets, des bonbons, de la confiserie, du chewing-gum, du tabac à chiquer, des cigarettes, des préparations pharmaceutiques, des produits de soins dentaires, tels que des gels et des pâtes dentifrice, des eaux dentifrice, des gargarismes, des produits de soins corporels et capillaires, tels que des shampoings, des gels de douche ou de bain, des déodorants et antiperspirants corporels, des lotions et baumes après-rasage, des mousses à raser, des parfums, etc.

Les proportions dans lesquelles les composés de l'invention peuvent être incorporés dans les produits divers susmentionnés varient dans une gamme de valeurs étendue. Ces valeurs sont dépendantes de la nature de l'article ou produit auquel on veut conférer un effet rafraîchissant et de l'effet recherché, ainsi que de la nature des coingrédients dans une composition donnée lorsque les composés de l'invention sont utilisés en mélange avec des coingrédients aromatisants ou parfumants, des solvants ou des adjuvants d'usage courant dans l'art.

A titre d'exemple, on peut citer des concentrations typiques de l'ordre de 0,001 à 5%, voire plus, de préférence de 0,002 à 1 % en poids de ce composé, par rapport au poids du produit fini rafraîchissant dans lequel il est incorporé.

Il convient de noter que les concentrations en composés de l'invention utilisées dans ces applications dépendent à la fois du produit à aromatiser et de l'effet recherché. Ainsi par exemple, dans des applications telles que boissons et bonbons, on utilisera typiquement des concentrations de l'ordre de 0.005 à 0.1 %, alors que pour l'aromatisation des dentifrices et chewing gums, les composés de l'invention seront typiquement utilisés à des concentrations comprises entre 0.2-0.3 et 0.5-1 %.

Les synthèses qui se prêtent pour obtenir les produits de formule (I) utilisent toutes le menthol en tant que produit de départ. L'une de ces synthèses possibles consiste en une estérification dudit menthol avec l'acide acétique qui est substitué en position α, à savoir un composé de formule 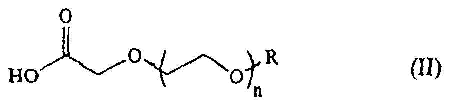
dans laquelle n et R ont les significations données dans la formule (I). La réaction se déroule sous catalyse d'un acide, comme par exemple l'acide p-toluènesulfonique, l'acide phosphorique ou tout acide connu pour ce type d'estérification. De préférence, la réaction est exécutée dans un solvant qui permet la séparation de l'eau formée par distillation azéotropique, comme par exemple le toluène, le benzène ou le xylène.

Une autre synthèse comprend, dans la première étape, l'estérification du menthol avec l'acide acétique α-halogéné, de préférence de l'acide 2-bromoacétique. Cette estérification est exécutée dans des conditions similaires à celles décrites dans le paragraphe précédent. Le 2-halogénoacétate de menthyle ainsi obtenu est ensuite transformé en le produit souhaité par une réaction d'éthérification dite de "Williamson". Dans cette réaction, on fait réagir un alcoolate d'un composé de formule 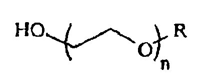
dans laquelle n et R ont les significations données dans la formule (I), avec le 2-halogénoacétate de menthyle susmentionné.

En tant qu'alcoolate, on peut utiliser l'alcoolate d'un métal alcalin, de préférence du sodium. Pour la réaction de Williamson, on utilise de préférence un solvant polaire aprotique tel que, par exemple, le diméthylformamide.

Après la réaction, les composés de formule (I) sont isolés et purifiés par des techniques courantes, telles que par exemple distillation ou chromatographie.

L'invention sera maintenant décrite de façon plus détaillée dans les exemples suivants, dans lesquels la température est indiquée en degrés Celsius et les abréviations ont le sens usuel dans l'art.

### Exemple 1

### Préparation du méthoxyacétate de (1R,3R,4S)-3-menthyle

On porte à reflux pendant 3 heures une solution de (-)-menthol (50 g, 320 mmole), d'acide méthoxyacétique (29 g, 320 mmole) et d'acide p-toluène sulfonique monohydraté (5 g, 26 mmole) dans 500 ml de toluène. L'eau dégagée lors de la réaction est séparée par distillation azéotropique. On lave 3 fois avec 100 ml d'une solution de NaOH à 5%, puis 2 fois avec 100 ml de saumure, sèche le solvant organique sur Na₂SO₄ et évapore le solvant sous vide. On distille ensuite sous vide, pour recueillir 51 g (81 %) d'un liquide incolore (point d'ébullition 75°C/3 Pa) ayant une pureté >99%.
SM(IE) : 83(100), 45(52), 55(48), 69(36), 139(28), 41(26), 97(19), 95(18), 29(14), 123(6), 155(1), 185(1), 213(1)
¹H-RMN (400MHz, CDCl₃) : 4,81 (dt, J=11,0, 4,4Hz, 1H)) ; 4,04, 3,98 (AB, 2H) ; 3,45 (s, 3H) ; 2,02, 1,99 (2m, 1H); 1,82 (m, 1H);1,71, 1,68 (2m, 2H) ; 1,50 (m, 1H) ; 1,40 (m, 1H) ; 1,14-0,82 (série de multiplets, 3H) ; 0,91 (d, J=5,6Hz, 3H) ; 0,89 (d, J=6,8Hz, 3H) ; 0,77 (d, J=6,8Hz, 3H) δ ppm
¹³C-RMN (90MHz, CDCl₃): 169.8 (s) ; 74,8 (d) ; 70,0 (t) ; 59,3 (q) ; 47,0 (d) ; 40,9 (t) ; 34,2 (t) ; 31,4 (d) ; 26,3 (d) : 23,4 (t) ; 22,0 (q) ; 20,7 (q) ; 16,3 (q) δ ppm

### Exemple 2

### Préparation de 3,6-dioxaheptanoate de (1R,3R,4S)-3-menthyle

On porte à reflux pendant 3 heures une solution de (-)-menthol (5 g, 32 mmole), d'acide (2-méthoxyéthoxy) acétique (4,3 g, 32 mmole) et d'acide p-toluène sulfonique monohydraté (0,5 g, 2,63 mmole) dans 50 ml de toluène. L'eau dégagée lors de la réaction est séparée par distillation azéotropique. On rajoute 150 ml de toluène, lave 3 fois avec 50 ml d'une solution de NaOH à 5%, puis 2 fois avec 50 ml de saumure, sèche le solvant organique sur Na₂SO₄ et évapore le solvant sous vide. On distille ensuite sous vide, pour recueillir 6,47 g (74%) d'un liquide incolore (point d'ébullition 110°C/3 Pa) ayant une pureté de 98%.
SM(IE) : 83(100), 55(45), 59(38), 69(36), 138(28), 45(23), 97(19), 29(16), 123(6), 109(3), 196(2), 155(1)
¹H-RMN (400MHz, CDCl₃) : 4,78 (dt, J=10,4, 4,4Hz, 1H) ; 4,16, 4,08 (AB, 2H) ; 3,72, 3,50 (2m, 4H) ; 3,40 (s, 3H) ; 2,02, 1,99 (2m, 1H) ; 1,82 (m, 1H) ; 1,71, 1,68 (2m, 2H) ; 1,50 (m, 1H) ; 1,39 (m, 1H) ; 1,14-0,82 (série de multiplets, 3H) ; 0,92 (d, J=6,3Hz, 3H) ; 0,89 (d, J=6,8Hz, 3H) ; 0,77 (d, J=6,8Hz, 3H) δ ppm
¹³C-RMN (90MHz, CDCl₃) : 170,0 (s) ; 74,8 (d) ; 71,9(t) ; 70,7 (t) ; 68,8 (t) ; 59,0 (q) ; 47,0 (d) ; 40,9 (t) ; 34,2 (t) : 31,4 (d) ; 26,3 (d) ; 23,4 (t) ; 22,0 (q) ; 20,7 (q) ; 16,3 (q) δ ppm

### Exemple 3

### Préparation de 3,6,9-trioxadécanoate de (1R,3R,4S)-3-menthyle

On porte à reflux pendant 3 heures une solution de (-)-menthol (5 g, 32 mmole), d'acide [2-(2-méthoxyéthoxy) éthoxy]acétique (5,7 g, 32 mmole) et d'acide p-toluène sulfonique monohydraté (0,6 g, 3,2 mmole) dans 20 ml de toluène. L'eau dégagée lors de la réaction est séparée par distillation azéotropique. On ajoute 150 ml de toluène, lave 3 fois avec 50 ml d'une solution de NaOH à 5%, puis 2 fois avec 50 ml de saumure, sèche le solvant organique sur Na₂SO₄ et évapore le solvant sous vide. On distille ensuite sous vide, pour recueillir 8,61 g (80%) d'un liquide incolore (point d'ébullition 160°C/1 Pa) ayant une pureté de 96%.
SM(lE) : 83(100), 59(74), 55(45), 103(44), 69(36), 138(34), 45(34), 95(22), 29(22), 133(16), 147(10), 178(8), 284(1)
¹H-RMN (400MHz, CDCl₃) : 4,78 (dt, J=10,4, 4,4Hz, 1H) ; 4,15, 4,09 (AB, 2H) ; 3,75, 3,71, 3,66, 3,55 (4m, 4H) ; 3,38 (s, 3H) ; 2,02, 1,99 (2m, 1H) ; 1,82 (m, 1H) ; 1,71, 1,68 (2m, 2H) ; 1,50 (m, 1H) ; 1,39 (m, 1H) ; 1,13-0,82 (série de multiplets, 3H) ; 0,92 (d, J=6,3Hz, 3H) ; 0,89 (d, J=6,8Hz, 3H) ; 0,76 (d, J=6,8Hz, 3H) δ ppm
¹³C-RMN (90MHz, CDCl₃) : 170.1(s) ; 74,8(d) ; 72,0, 70,9, 70,64, 70,58, 68,8(5t) ; 59,1 (q) ; 47,0(d) ; 40,9(t) ; 34,2(t) ; 31,4(d) ; 26,3(d) ; 23,5(t) ; 22,0(q) ; 20,7(q) ; 16,3(q) δ ppm

### Exemple 4

### Préparation de 3,6,9,12,15-pentaoxahexadécanoate de (1R,3R,4S)-3-menthyle

Ce produit a été synthétisé en deux étapes :
a) Pour la première étape, on a porté à reflux pendant 3 heures une solution de (-)-menthol (1,56 g, 10 mmole), d'acide 2-bromoacétique (1,39 g, 10 mmole) et d'acide p-toluène sulfonique monohydraté (0,5 g, 2,6 mmole) dans 100 ml de toluène. L'eau dégagée lors de la réaction est séparée par distillation azéotropique. On ajoute 100 ml de toluène et on lave 3 fois avec 10 ml d'une solution de NaOH à 5%, puis 2 fois avec 100 ml de saumure, sèche le solvant organique sur Na₂SO₄ et évapore le solvant sous vide. On obtient le 2-bromoacétate de menthyle (2,45 g, 89%) qui est utilisé sans purification pour l'étape suivante.
b) La seconde étape consiste en une réaction dite de "Williamson".

On génère l'alcoolate du tétraéthylèneglycol monométhyl éther (1,87 g, 9 mmole) en chauffant ledit alcool avec du sodium (0,23 g, 10 mmole) dans le diméthylformamide (20 ml) à 60°C durant 4 heures. On ajoute ensuite le bromoacétate de menthyle obtenu précédemment (2,45 g, 9 mmole) et laisse agiter 3 h à 60-80°C, puis 16 h à température ambiante. On ajoute 200 ml d'acétate d'éthyle, lave 3 fois avec 20 ml d'une solution de KHSO₄ à 5%, puis 3 fois avec 20 ml d'une solution de NaOH à 5%, et ensuite 2 fois avec 20 ml de saumure. On sèche le solvant organique sur Na₂SO₄ et évapore le solvant sous vide. Le produit est purifié par flash-chromatographie sur gel de silice élué avec un mélange cyclohexane/acétate d'éthyle (60/40). On obtient 0,5 g d'une huile légèrement jaune (14%). La réaction n'a pas été optimisée.
SM(IC, NH₃) : 422(100, M⁺ NH₄⁺), autres fragments minimes
¹H-RMN (400MHz, CDCl₃) : 4,78 (dt, J=10,4, 4,4Hz, 1H) ; 4,15, 4,08 (AB, 2H) ; 3,72-3,50 (série de m, 24H) ; 3,38 (s, 3H) ; 2,02,1,99 (2m, 1H) ; 1,82 (m, 1H) ; 1,71, 1,68 (2m, 2H) ; 1,50 (m, 1H) ; 1,39 (m, 1H) ; 1,14-0,82 (série de multiplets, 3H) ; 0,92 (d, J=6,3Hz, 3H) ; 0,89 (d, J=6,8Hz, 3H) ; 0,77 (d, J=6,8Hz, 3H) δ ppm
¹³C-RMN (90MHz, CDCl₃): 170.1 (s); 74,8(d) ; 72,0(t) ; série de triplets à 72,0(t) ; 70,9(t) ; 70,6(t) ; 70,5(t) et 68,8(t) ; 59,0(q) ; 47,0(d) ; 40,9(t) ; 34,2(t) ; 31,4(d) ; 26,3(d) ; 23,4(t) ; 22,0(q) ; 20,7(q) ; 16,3(q) δ ppm

### Exemple 5

### Préparation du (2-hydroxyéthoxy)acétate de (1R, 3R, 4S)-menthyle

Ce produit a été synthétisé en trois étapes :
a) Pour la première étape, on ajoute, par portions, du NaH (60% dans de l'huile minérale, 1.97 g, 1.5 eq) à une solution de 2-benzyloxyéthanol (5 g, 32.85 mmole ; origine : Aldrich Chemicals) dans 25ml de THF sous argon.
   On porte la solution résultante à reflux pendant 1 heure, puis on la refroidit à 25°.
   On prépare une solution de bromoacétate de sodium en ajoutant du NaH par portions (1.3g, 1 eq) à une solution d'acide bromoacétique (4.56 g, 32.85 mmole) dans 25 ml de THF.
   On ajoute goutte à goutte cette solution de bromoacétate à la solution préparée d'anion benzyloxyéthanolate et on chauffe le mélange à reflux pendant 19 heures. On éteint la réaction avec du KHSO₄ à 5%. On ajoute 250 ml d'acétate d'éthyle et on extrait la phase organique 3 fois à l'aide de NaOH à 5%. On acidifie la phase alcaline à pH 1 avec de l'HCl à 10% et on extrait 2 fois avec EtOAc. On lave la phase organique 3 fois avec de la saumure, on sèche sur Na₂SO₄ et on évapore pour obtenir 6.4 g (93%) d'acide 7-phényl-3,6-dioxaheptanoïque sous forme de liquide incolore.
b) On chauffe à reflux azéotropiquement pendant 3 heures un mélange d'acide 7-phényl-3,6-dioxaheptanoïque (6.36 g, 30.3 mmole), (-)menthol (4.73 g, 1 eq) et acide p-toluènesulfonique (0.63 g, 0.11 eq.) dans 40 ml de toluène.
   On ajoute de l'acétate d'éthyle et on lave 3 fois la phase organique à l'aide de NaHCO₃ à 5%, trois fois avec de la saumure, puis on séche sur Na₂SO₄ et évapore pour obtenir 10.9 g d'une huile jaune pâle que l'on distille sous vide pour obtenir 8.96 g (85%, p.e. 145-147°/10-2 mmHg) d'une huile incolore correspondant au 7-phényl-3,6-dioxaheptanoate de (1R, 3R, 4S)-menthyle.
c) On hydrolyse le 7-phényl-3,6-dioxaheptanoate de (1R, 3R, 4S)-menthyle (8.9 g, 25.5 mmole) durant 16 heures dans 50 ml de THF, à l'aide de Pd sur charbon de bois à 10% (0.89 g) comme catalyseur. On ajoute de l'acétate d'éthyle puis on filtre le mélange réactionnel sur lit de célite, on lave trois fois à la saumure, on sèche sur Na₂SO₄ et on évapore. On effectue une chromatographie flash sur gel de silice (cyclohexane/EtOAc ; 80/20) pour obtenir le (2-hydroxyéthoxy)acétate de (1R, 3R, 4S)-menthyle sous forme d'un liquide jaune pâle (5.37 g ; 89.8%). Pureté : 99.2% par chromatographie en phase gazeuse (colonne DB1, 15m, 70° pour 0.5 min. puis 70-220° à 10°/min., T. R = 11.76 min.).

SM(EI) : 83(100), 81(86), 95(85), 71(64), 138(51), 123(50), 55(43), 41(24), 102(19), 109(16), 29(12), 155(2)
¹H-RMN (CDCl₃) : 4,81 (dt, J=10.7, 4,4Hz, 1H) ; 4,16-4,09 (AB, J=16,7Hz, 2H) ; 3,76 (m, 2H) ; 3,69 (m, 2H) ; 3,06 (s large, 1H, OH échangeable) ; 2,03-2,00 (2m, 1H) ; 1,83 (m, 1H) ; 1,72-1,68 (2m, 2H) ; 1,52 (m, 1H) ; 1,44-1,37 (m, 1H) ; 1,09-0,83 (m, 3H) ; 0,93 ; 0,91 (2d, J=6,8Hz, 6H) ; 0,78 (d, J=7,1 Hz, 3H) δ ppm
¹³C-RMN (CDCl₃) : 170,8(s) ; 75,4(d) ; 73,6(t) ; 68,5(t) ; 61,5(t) ; 47,0 (d) ; 40,9(t) ; 34,1 (t); 31,4(d) ; 26,4(d) ; 23,5(t) ; 22,0(q) ; 20,7(q) ; 16,3(q) δ ppm
Goût : le (2-hydroxyéthoxy)acétate de (1R, 3R, 4S)-menthyle présente un côté rafraîchissant immédiat et intense lorsque goûté.

### Exemple 6

### Préparation du 11-hydroxy-3,6,9-trioxaundécanoate de (1R, 3R, 4S)-menthyle

Ce produit a été synthétisé en deux étapes :
a) Pour la première étape, on chauffe du (-)-menthol (20 g ; 127 mmole) et de l'acide 3,6,9-trioxaundécanoique (56.9g, 2eq. ; origine : Fluka), sans solvant, à 120° pendant 16 heures en retirant continuellement l'eau par distillation à 10 mmHg. On dilue le mélange réactionnel dans de l'acétate d'éthyle et on lave 8 fois à l'eau désionisée, on sèche sur Na₂SO₄ et on évapore. On effectue une chromatographie du produit brut sur gel de silice (cyclohexane/EtOAc, 65/35 avec 1 % d'acide acétique, puis 40/60 avec 1 % d'éthanol) pour obtenir 25 g de hydrogéno-3,6,9-trioxaundécanedioate de (1R, 3R, 4S)-menthyle (54%) sous forme d'un liquide visqueux incolore.
b) On ajoute goutte à goutte un complexe borane-tétrahydrofurane (1 M, 20 ml) à une solution de hydrogéno-3,6,9-trioxaundécanedioate de (1R, 3R, 4S)-menthyle (7.21 g ; 20 mmole) dans 72 ml de THF à température ambiante. On maintient la réaction sous agitation pendant 4.5 heures, puis on refroidit à 0° et on ajoute 5ml de NaOH 6N goutte à goutte. On maintient sous agitation pendant 10 min. supplémentaires. On ajoute de l'acétate d'éthyle et on lave la phase organique 3 fois à l'aide de NaHCO₃ à 5%, 3 fois avec KHSO₄ à 5%, 3 fois à la saumure, on sèche sur Na₂SO₄ et on évapore. On effectue une chromatographie flash sur gel de silice (cyclohexane/EtOAc ; 40/60) pour obtenir le 11-hydroxy-3,6,9-trioxaundécanoate de (1R, 3R, 4S)-menthyle sous forme de liquide jaune pâle (2.22g, 32%). Pureté : 99.9% par chromatographie en phase gazeuse (colonne DB1, 15m, 150° pendant 15 min. puis 150-240° à 10°/min., T.R. = 9.17 min.).

SM(El): 83(100), 45(52), 89(46), 138(45), 103(39), 69(31), 55(30), 147(15), 121(15), 208(7), 163(6), 190(6), 177(5)
¹H-RMN (CDCl₃): 4,78 (dt, J=10,7, 4,4Hz, 1H); 4,16 ; 4,09 (AB, J=16,7Hz, 2H) ; 3,76-3,61 (m, 12H) ; 2,78 (s large, 1H, OH échangeable) ; 2,02 ; 1,98 (2m, 1H) ; 1,83 (m, 1H) ; 1,70 ; 1,66 (2m, 2H) ; 1,49 (m, 1H) ; 1,42-1,34(m, 1H); 1,01-0,80 (m, 3H) ; 0,93 ; 0,91 (2d, J=5,9Hz, 6H) ; 0,78 (d, J=6,7Hz, 3H) δ ppm
¹³C-RMN (CDCl₃) : 170,1(s); 75,0(d); 72,7(t); 70,7; 70,6 ; 70,5 ; 70,2 (4t) ; 68,7(t); 61,6(t) ; 47,0(d) ; 40,9(t); 34,2 (t) ; 31,4(d) ; 26,3(d) ; 23,4(t) ; 22,0(q); 20,7(q); 16,3(q) δ ppm
Goût : légèrement amer, le 11-hydroxy-3,6,9-trioxaundécanoate de (1R, 3R, 4S)-menthyle a un goût mentholé. Son effet rafraîchissant se développe plus spécialement sur la langue et dans la gorge.

### Exemple 7

### Préparation d'un gel et d'une pâte dentifrice

Le méthoxacétate de (1R,3R,4S)-3-menthyle et le 3,6-dioxaheptanoate de (1R,3R,4S)-3-menthyle ont été dosés à 0,4% chacun dans un gel et une pâte dentifrice de type courant, que l'on a préparés par exemple à partir des ingrédients suivants :

### Gel dentifrice :

| Ingrédients | % en poids |
|---|---|
| Sorbosil®AC 77¹) | 8 |
| Sorbosil® TC 15¹) | 9 |
| Sorbitol à 70% | 66,642 |
| PEG 1500 | 2 |
| Sel sodique de lauryl-sulfate | 2,1 |
| Sel sodique de monofluorophosphate | 0,76 |
| Sel sodique de carboxyméthylcellulose | 0,4 |
| Sel sodique de saccharine | 0,2 |
| Colorant bleu | 0,002 |
| Eau déminéralisée | 10,896 |
| Total | 100,00 |

### Pâte dentifrice :

| Ingrédients | % en poids |
|---|---|
| Sorbosil AC 77¹) | 6,5 |
| Sorbosil TC 15¹⁾ | 9 |
| Sorbitol à 70% | 40 |
| Sel sodique de lauryl-sulfate | 1,5 |
| Sel sodique de monofluorophosphate | 0,8 |
| Sel sodique de carboxyméthylcellulose | 1,1 |
| Sel sodique de saccharine | 0,2 |
| TiO₂ | 0,5 |
| Eau déminéralisée | 40,4 |
| Total | 100,00 |

| | |
|---|---|
| 1) agent épaississant à base de silice ; origine : Crosfield Chemicals Ltd, Grande-Bretagne | |

Les produits ont ensuite été testés et évalués à l'aveugle par des experts aromaticiens. Après l'utilisation et le rinçage de la bouche, on a constaté, pour chacun de ces produits, une fraîcheur qui s'est développée après le rinçage et qui a duré environ 15 à 20 minutes. Lorsque comparés au menthol, il a été jugé que les produits de l'invention cités plus haut apportaient une fraîcheur qui avait un effet retard par rapport à celle du menthol et, de plus, on n'apercevait pas le goût typique de celui-ci.

En appliquant un mélange des deux composés mentionnés auparavant à un dosage de 0,25% chacun dans le produit fini, on a obtenu un effet très apprécié dans ce type d'application en diminuant à la fois la note fruitée du méthoxyacétate de (1R,3R,4S)-3-menthyle et la note amère du 3,6-dioxaheptanoate de (1R,3R,4S)-3-menthyle, par rapport aux produits décrits plus haut qui ne contenaient que l'un ou l'autre de ces composés.

### Exemple 8

### Préparation de bonbons

On a préparé des bonbons au goût de pamplemousse à partir de sucre cuit, 1% d'acide citrique et 0,05% d'un arôme pamplemousse de formule suivante :

| Ingrédients | Parties en poids |
|---|---|
| Acétate de styrallyle | 25 |
| Thiomenthone à 0,1 % dans l'éthanol | 30 |
| Essence de pamplemousse | 945 |
| Total | 1000 |

Ces bonbons, sans aucune adjonction, ont ensuite été comparés à l'aveugle par des experts aromaticiens aux bonbons de la même composition auxquels on avait ajouté certains composés selon l'invention.

De l'opinion des aromaticiens, l'adjonction de 0,05% du méthoxyacétate de (1R,3R,4S)-3-menthyle a apporté une fraîcheur qui ne modifie pas le profil organoleptique de la composition de base, à savoir les bonbons décrits ci-dessus.

L'adjonction de la même quantité de 3,6-dioxaheptanoate de (1R,3R,4S)-3-menthyle a apporté une fraîcheur similaire et, de plus, une amertume qui a renforcé l'amertume naturelle de l'essence de pamplemousse.

### Exemple 9

### Préparation de confiseries à base de gélatine

On a préparé de façon connue en soi des confiseries au goût de pamplemousse à partir de 30 g de gélatine, 175 g d'eau, 150 g de sucre et 200 g de glucose. On a ensuite rajouté 0,8% d'acide citrique et 0,08% d'un arôme pamplemousse selon la formule donnée à l'Exemple 6.

Cette confiserie de base, sans aucune adjonction, a ensuite été comparée à l'aveugle par des experts aromaticiens à la confiserie de la même composition à laquelle on avait ajouté certains composés de l'invention.

De l'avis des aromaticiens, l'adjonction de 0,05% en poids du méthoxyacétate de (1R,3R,4S)-3-menthyle à la confiserie de base laisse une fraîcheur en bouche. L'adjonction de 0,05% de 3,6-dioxaheptanoate de (1R,3R,4S)-3-menthyle a le même effet de fraîcheur, tout en renforçant aussi l'amertume de l'arôme pamplemousse.

### Exemple 10

### Préparation de sorbets citron

On a préparé des sorbets citron à partir des ingrédients suivants, en utilisant des techniques courantes :

| Ingrédients | % en poids |
|---|---|
| Sucre | 20 |
| Sirop de glucose | 8 |
| Dextrose | 2,5 |
| Concentré de jus de citron | 1,5 |
| Meypyrogen IC 304 ¹⁾ | 0,6 |
| Eau | à 100% |
| Total | 100 |

| | |
|---|---|
| 1) mélange de gomme de caroube E410, gomme de guar E412, carrageenane E407, gélatine, émulsifiant E471 ; origine : Meyhall Chemical AG, Kreuzlingen, Suisse | |

On a ensuite rajouté 0,5% d'acide citrique et 0,01 % d'arôme citron de formule suivante :

| Ingrédients | Parties en poids |
|---|---|
| Acétate de citronellyle | 2 |
| Acétate de géranyle | 6 |
| Acétate de linalyle | 2 |
| Citronellol | 2 |
| Géraniol | 3 |
| Terpinéol | 5 |
| Citral | 5 |
| Terpènes de citron | 975 |
| Total | 1000 |

Ce sorbet, sans aucune adjonction, a ensuite été comparé à l'aveugle par des experts aromaticiens aux sorbets de la même composition auxquels on avait ajouté certains composés selon l'invention.

De l'opinion des aromaticiens, l'adjonction de 0,05% en poids de méthoxyacétate de (1R,3R,4S)-3-menthyle au sorbet de base préparé ci-dessus lui a apporté une fraîcheur agréable dans la bouche.

L'adjonction de 0,003% de 3,6-dioxaheptanoate de (1R,3R,4S)-3-menthyle a un effet similaire, mais ce composé apporte en plus une amertume qui renforce la note zeste de l'arôme citron.

### Exemple 11

### Préparation de chewing-gum

On a préparé du chewing-gum à partir d'une base de chewing-gum de type Cafosa Nevada Plus T 413-01 (18 parties en poids) (origine : Cafosa Gum Products Technology, Barcelone, Espagne), du sucre (60 parties en poids), du glucose (20 parties en poids) et du glycérol (0,5 parties en poids). On a ensuite rajouté à ce mélange 0,8 parties en poids d'acide citrique et 1 partie en poids d'arôme citron selon la formule donnée à l'Exemple 8. Ce chewing-gum de base, sans aucune adjonction, a ensuite été comparé à l'aveugle par des experts aromaticiens aux chewing-gums de la même composition auxquels on avait ajouté certains composés selon l'invention.

Ainsi, l'adjonction de 0,4 parties en poids chacun de méthoxyacétate de (1R,3R,4S)-3-menthyle ou de 3,6-dioxaheptanoate de (1R,3R,4S)-3-menthyle au chewing-gum lui confère un effet de fraîcheur prolongé, la sensation de fraîcheur s'installant en bouche après la mastication.

### Exemple 12

### Préparation d'une boisson à l'orange

On a préparé une boisson à l'arôme orange à partir d'un sirop 65°B qui a été dilué à 10%, acidifié avec 1,5% d'acide citrique et ensuite aromatisé avec 0,01% d'un arôme orange de formule suivante :

| Ingrédients | Parties en poids |
|---|---|
| Hexanal | 3 |
| Octanal | 2 |
| Dodécanal | 3 |
| Butyrate d'éthyle | 15 |
| Aldéhyde acétique | 30 |
| Essence d'orange | 947 |
| Total | 1000 |

Cette boisson de base, sans aucune adjonction, a ensuite été comparée à l'aveugle par des experts aromaticiens aux boissons de la même composition auxquelles on avait ajouté certains composés selon l'invention.

De l'opinion des aromaticiens, l'adjonction de 0,003% de méthoxyacétate de (1R,3R,4S)-3-menthyle donne une sensation de fraîcheur qui se développe en après-goût.

L'adjonction de 0,003% de 3,6-dioxaheptanoate de (1R,3R,4S)-3-menthyle a le même effet, mais ce composé apporte en plus une amertume qui renforce la note zeste de l'arôme.

### Exemple comparatif 1

### Préparation de lotions après-rasage

On a préparé deux lotions après-rasage de façon connue en soi à partir des ingrédients suivants :

| | | Ingrédients | % en poids |
|---|---|---|---|
| A | 1) | Cremophor RH-40 ¹⁾ | 1,5 |
| | 2) | Alcool éthylique à 10% | 98,0 |
| | 3) | Frescolat® type ML ²⁾ | 0,5 |

| | | | |
|---|---|---|---|
| 1) huile de ricin hydrogéné et éthoxylé ; origine : BASF AG, Ludwigshafen, Allemagne | | | |
| 2) lactate de (1R,3R,4S)-3-menthyle; origine: Haarmann & Reimer GmbH, Holzminden, Allemagne | | | |

| | | Ingrédients | % en poids |
|---|---|---|---|
| B | 1 ) | Cremophor RH-40 ¹) | 1,5 |
| | 2) | Alcool éthylique à 10% | 98,0 |
| | 3) | 3,6-dioxaheptanoate de (1R,3R,4S)-3-menthyle | 0,5 |

| | | | |
|---|---|---|---|
| 1) voir lotion A | | | |

Les deux lotions ainsi obtenues ont été versées dans un flacon de type aérosol.

Elles ont ensuite été appliquées dans une quantité de 300 mg sur l'avant-bras et la joue de chacune des deux personnes formant le panel et comparées à l'aveugle.

De l'opinion du panel, la lotion contenant le composé selon l'invention a montré un effet rafraîchissant supérieur à celui de la lotion contenant l'agent rafraîchissant de l'art antérieur dans 3 sur 4 essais réalisés.

## Revendications

1. Composé de formule 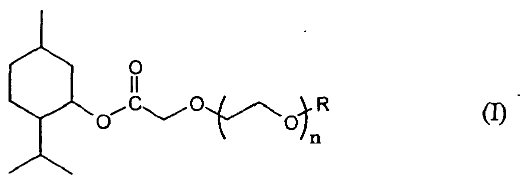
dans laquelle R=H ou CH₃ et n est un nombre entier de 1 à 4.

2. Composé selon la revendication 1, **caractérisé en ce qu'**il se présente sous forme de l'isomère de configuration (1R,3R,4S).

3. Le 3,6-dioxaheptanoate de (1R,3R,4S)-3-menthyle.

4. Mélange de méthoxyacétate de (1R,3R,4S)-3-menthyle et de 3,6-dioxaheptanoate de (1R,3R,4S)-3-menthyle.

5. Mélange selon la revendication 4, **caractérisé en ce que** chacun des deux composés mentionnés est présent dans une proportion de 50% en poids.

6. Utilisation en tant qu'agent rafraîchissant d'un composé de formule 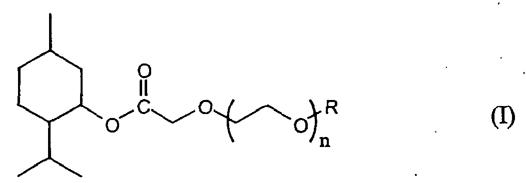
dans laquelle R=H ou CH₃ et n est un nombre entier de 1 à 4.

7. Utilisation selon la revendication 6, **caractérisée en ce que** le composé se présente sous forme de l'isomère de configuration (1R,3R,4S).

8. Utilisation en tant qu'agent rafraîchissant du 3,6-dioxaheptanoate de (1R,3R,4S)-3-menthyle.

9. Utilisation en tant qu'agent rafraîchissant d'un mélange de méthoxyacétate de (1R,3R,4S)-3-menthyle et de 3,6-dioxaheptanoate de (1R,3R,4S)-3-menthyle.

10. Utilisation d'un mélange selon la revendication 9, **caractérisée en ce que** chacun des deux composés mentionnés est présent dans une proportion de 50% en poids.

11. Composition aromatisante ou produit aromatisé, contenant à titre d'ingrédient un composé ou un mélange selon l'une des revendications 1 à 5.

12. Produit aromatisé selon la revendication 11, sous forme d'une boisson, en particulier d'un jus de fruits, d'une limonade ou d'un thé froid, d'une glace ou sorbet, d'un bonbon, d'une confiserie, d'un chewing-gum, d'une cigarette, de tabac à chiquer, d'une préparation pharmaceutique, d'un produit de soin dentaire ou de soin corporel, en particulier d'un gel ou d'une pâte dentifrice, d'une eau dentifrice ou d'un gargarisme.

13. Article parfumé, contenant à titre d'ingrédient an composé ou un mélange selon l'une des revendications 1 à 5.

14. Article parfumé selon la revendication 13, sous forme d'un shampoing, d'un gel de douche ou de bain, d'un déodorant ou antiperspirant corporel, d'une lotion ou d'un baume après-rasage, d'une mousse à raser ou d'un autre produit cosmétique, ou d'un parfum.

15. Procédé de préparation d'un composé de formule (1) comme précisé dans la revendication 1, **caractérisé en ce que** le menthol est estérifié avec un dérivé de l'acide acétique de formule 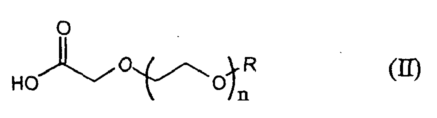
dans laquelle n et R ont les significations données dans la revendication 1.

16. Procédé de préparation d'un composé de formule (I) comme précisé dans la revendication 1, **caractérisé en ce que** :
a) le menthol est estérifié avec de l'acide acétique α-halogéné, de préférence de l'acide 2-bromoacétique, et
b) le 2-halogénoacétate de menthyle ainsi obtenu est ensuite éthérifié dans une réaction dite de "Williamson", à l'aide d'un alcoolate d'un composé de formule 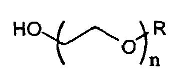 dans laquelle n et R ont les significations données dans la revendication 1.

## Patentansprüche

1. Verbindung der Formel 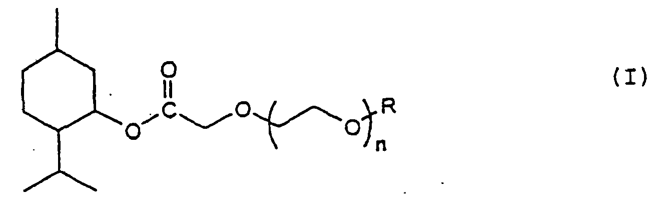 in der R=H oder CH₃ und n eine ganze Zahl von 1 bis 4 ist.

2. Verbindungen nach Anspruch 1, **dadurch gekennzeichnet, daß** sie in Form des Isomers mit der Konfiguration (1R,3R,4S) vorliegen.

3. (1R,3R,4S)-3-Menthyl-3,6-dioxaheptanoat.

4. Mischung aus (1R,3R,4S)-3-Menthyl-methoxyacetat und (1R,3R,4S)-3-Menthyl-3,6-dioxaheptanoat.

5. Mischung nach Anspruch 4, **dadurch gekennzeichnet, daß** jede der beiden genannten Verbindungen in einem Anteil von 50 Gew.-% vorliegt.

6. Verwendung als Erfrischungsmittel einer Verbindung der Formel 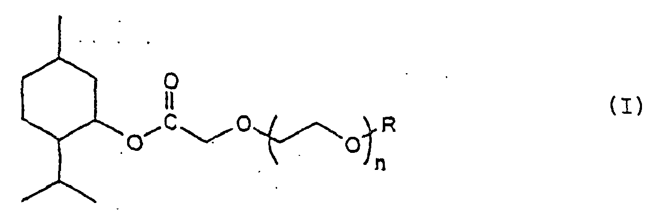 in der R=H oder CH₃ und n eine ganze Zahl von 1 bis 4 ist.

7. Verwendung nach Anspruch 6, **dadurch gekennzeichnet, daß** die Verbindungen in Form des Isomers mit der Konfiguration (1R,3R,4S) vorliegen.

8. Verwendung als Erfrischungsmittel von (1R,3R,4S)-3-Menthyl-3,6-dioxaheptanoat.

9. Verwendung als Erfrischungsmittel einer Mischung aus (1R,3R,4S)-3-Menthyl-methoxyacetat und (1R,3R,4S)-3-Menthyl-3,6-dioxaheptanoat.

10. Verwendung einer Mischung nach Anspruch 9, **dadurch gekennzeichnet, daß** jede der beiden genannten Verbindungen in einem Anteil von 50 Gew.-% vorliegt.

11. Aromatisierende Zusammensetzung oder aromatisiertes Produkt, welche(s) als Inhaltsstoff eine Verbindung oder eine Mischung nach einem der Ansprüche 1 bis 5 enthält.

12. Aromatisiertes Produkt nach Anspruch 11 in Form eines Getränks, insbesondere eines Fruchtsaftes, einer Limonade oder eines kalten Tees, eines Speiseeises oder Sorbets, eines Bonbons, einer Süßware, eines Kaugummis, einer Zigarette, eines Kautabaks, eines pharmazeutischen Präparats, eines Zahn- oder Körperpflegeproduktes, insbesondere eines Zahngels oder einer Zahnpaste, eines Mund- oder Gurgelwassers.

13. Parfümierter Artikel, welcher als Inhaltsstoff eine Verbindung oder eine Mischung nach einem der Ansprüche 1 bis 5 enthält.

14. Parfümierter Artikel nach Anspruch 13 in Form eines Shampoos, eines Dusch- oder Badegels, eines Körperdeodorants oder Antitranspirans, einer Rasierlotion oder eines Rasierbalsams, eines Rasierschaums oder eines anderen kosmetischen Produktes, oder eines Parfüms.

15. Verfahren zur Herstellung einer Verbindung der Formel (I) gemäß den Angaben in Anspruch 1, **dadurch gekennzeichnet, daß** das Menthol mit einem Essigsäurederivat der Formel 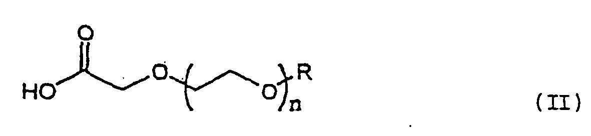 verestert wird, in der n und R die in Anspruch 1 angegebenen Bedeutungen haben.

16. Verfahren zur Herstellung einer Verbindung der Formel (I) gemäß den Angaben in Anspruch 1, **dadurch gekennzeichnet, daß**:
a) das Menthol mit α-halogenierter Essigsäure, vorzugsweise 2-Bromessigsäure, verestert wird, und
b) das auf diese Weise erhaltene Menthyl-2-halogen-acetat anschließend in einer sogenannten "Williamson"-Reaktion mit Hilfe eines Alkoholats einer Verbindung der Formel 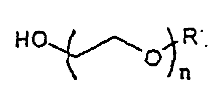 verethert wird, in der n und R die in Anspruch 1 angegebenen Bedeutungen haben.

## Claims

1. A compound of formula 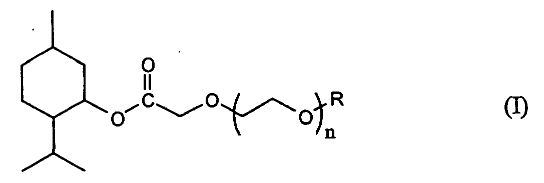 in which R = H or CH₃ and n is a whole number from 1 to 4.

2. A compound according to claim 1, **characterised in that** it is in the form of the isomer of configuration (1R,3R,4S).

3. (1R,3R,4S)-3-Menthyl 3,6-dioxaheptanoate.

4. A mixture of (1R,3R,4S)-3-menthyl methoxyacetate and (1R,3R,4S)-3-menthyl 3,6-dioxaheptanoate.

5. A mixture according to claim 4, **characterised in that** each of the two compounds mentioned is present in a proportion of 50% by weight.

6. Use, as a cooling agent, of a compound of formula 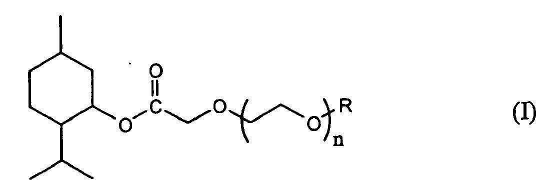 in which R = H or CH₃ and n is a whole number from 1 to 4.

7. Use according to claim 6, **characterised in that** the compounds are in the form of the isomer of configuration (1R,3R,4S).

8. Use of (1R,3R,4S)-3-menthyl 3,6-dioxaheptanoate as a cooling agent.

9. Use of a mixture of (1R,3R,4S)-3-menthyl methoxyacetate and (1R,3R,4S)-3-menthyl 3,6-dioxaheptanoate as a cooling agent.

10. Use of a mixture according to claim 9, **characterised in that** each of the two compounds mentioned is present in a proportion of 50% by weight.

11. A flavouring composition or flavoured product, containing as an ingredient a compound or a mixture according to one of claims 1 to 5.

12. A flavoured product according to claim 11, in the form of a drink, in particular a fruit juice, a soft drink or a cold tea, an ice cream or sorbet, a sweet, a preserve, a chewing gum, a cigarette, chewing tobacco, a pharmaceutical preparation, a dental-care or body-care product, in particular a dentifrice gel or paste, a mouth wash or a gargle.

13. A perfumed article, containing as an ingredient a compound or a mixture according to one of claims 1 to 5.

14. A perfumed article according to claim 13, in the form of a shampoo, a shower or bath gel, a body deodorant or antiperspirant, an after-shave lotion or balm, a shaving foam or another cosmetic product, or a perfume.

15. A process for preparing a compound of formula (I) as specified in claim 1, **characterised in that** the menthol is esterified with a derivative of acetic acid of formula 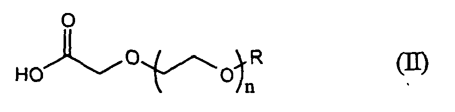 in which n and R have the meanings assigned in claim 1.

16. A process for preparing a compound of formula (I) as specified in claim 1,
**characterised in that**:
a) the menthol is esterified with α-halogenated acetic acid, preferably 2-bromoacetic acid, and
b) the menthyl 2-halogenoacetate thus obtained is then etherified in a "Williamson" reaction by means of an alcoholate of a compound of formula 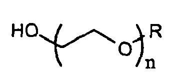
in which n and R have the meanings assigned in claim 1.
